# EUROPÄISCHE PATENTSCHRIFT

(11) **EP 2 947 426 B1**
(45) Veröffentlichungstag und Bekanntmachung des Hinweises auf die Patenterteilung: **12.09.2018**
(21) Anmeldenummer: 15168977.5
(22) Anmeldetag: 22.05.2015
(51) Int. Cl.: G01D 18/00, G01N 33/00

(54) **VERFAHREN ZUM BETREIBEN EINER MESSSTELLE**
METHOD FOR OPERATING A MEASURING POINT
PROCÉDÉ DE FONCTIONNEMENT D'UN POINT DE MESURE

(30) Priorität: 23.05.2014 DE 102014107275
(43) Veröffentlichungstag der Anmeldung: 25.11.2015
(73) Patentinhaber: Krohne Messtechnik GmbH, 47058 Duisburg (DE)
(72) Erfinder: Deilmann, Michael, 45257 Essen (DE); Schmits, Christoph, 44149 Dortmund (DE)
(74) Vertreter: Gesthuysen Patent- und Rechtsanwälte

(56) Entgegenhaltungen:
- WO-A2-2004/025223
- DE-A1-102007 053 223
- DE-A1-102010 062 657

## Beschreibung

Die Erfindung betrifft ein Verfahren zum Betreiben einer Messstelle, wobei an der Messstelle von mindestens einem kalibrierbaren Sensor mindestens eine Messgröße ermittelt wird. Weiterhin bezieht sich die Erfindung auf ein Verfahren zum Betreiben einer Zweit-Messstelle, wobei an der Zweit-Messstelle von mindestens einem Zweit-Sensor mindestens eine Messgröße ermittelt wird.

Im Bereich der Analysenmesstechnik werden die eingesetzten Messsensoren teilweise regelmäßig ausgetauscht, da sie einer natürlichen Alterung unterliegen. Zusätzlich können besondere Situationen auftauchen, die einen Austausch erforderlich machen. So kann zum Beispiel in einem pH-Sensor die Pufferlösung mit dem pH-Wert 7 verunreinigt werden oder die Elektrode der Sonde zeigt einen falschen Messwert an. Solche und auch andere Sensoren werden im Allgemeinen in regelmäßigen Abständen kalibriert.

Um eine Abschätzung über die Lebensdauer eines Sensors zu erhalten, ist es im Stand der Technik bekannt, aus Zustands- oder Prüfparametern auf das zukünftige Verhalten zu extrapolieren (siehe z. B. WO 2004/025223 A2).

Dies steht jeweils im Zusammenhang mit einer Messstelle, an denen ein Sensor wenigstens eine Messgröße ermittelt. Das Betreiben einer solchen Messstelle bezieht sich dabei auch auf die Durchführung bzw. Planung von Kalibrierungen oder das zeitnahe Austauschen der verwendeten Sensoren. Letzteres macht ein Wissen über die Rest-Lebensdauer des Sensors erforderlich.

Die Druckschrift DE 10 2010 062 657 A1 beschreibt ein Verfahren zur Bereitstellung von Kalibrierungsdaten für eine modulare Messeinrichtung. Dabei wird angegeben, dass Kalibrierungsdaten, die für einen Sensor gewonnen werden, für die Sensoren der gleichen Charge Verwendung finden. Zudem wird offenbart, dass der Vergleich von unterschiedlichen Kalibrierungsdaten Rückschlüsse auf das Alterungsverhalten zulassen, wobei das Alterungsverhalten abhängig von den Einsatzbedingungen und damit auch vom Einsatzort ist.

Aus der Druckschrift DE 10 2007 053 223 A1 ist ein Verfahren zum Betreiben einer Messstelle, wobei an der Messstelle von einem kalibrierbaren Sensor eine Messgröße ermittelt wird, bekannt. Die Sensoreinheit weist ein messstellenspezifisches Softwaremodul auf, das eine Prognose von Kalibrierzeiten und anderen Wartungsmaßnahmen ermöglicht.

Der Erfindung liegt daher die Aufgabe zugrunde, ein Verfahren zum Betreiben einer Messstelle einer Prozessanlage zu beschreiben, in der alternativ zum Stand der Technik insbesondere eine möglichst genaue Planung von Aktivitäten, wozu beispielsweise das Kalibrieren oder der Sensor-Tausch gehören, möglich ist.

Das erfindungsgemäße Verfahren zum Betreiben einer Messstelle, wobei an der Messstelle von mindestens einem kalibrierbaren Sensor mindestens eine Messgröße ermittelt wird, wobei der Sensor an vorgebbaren Kalibrier-Zeitpunkten kalibriert wird, und wobei jeweils mindestens ein mit der Kalibrierung verbundener Parameter als Teil von Referenzdaten des Sensors hinterlegt wird und bei dem die zuvor hergeleitete und aufgezeigte Aufgabe gelöst ist, ist zunächst und im Wesentlichen dadurch gekennzeichnet, dass , an einer Zweit-Messstelle von einem Zweit-Sensor mindestens eine Messgröße ermittelt wird, dass ausgehend von den Referenzdaten des Sensors mindestens eine von der Alterung abhängige Größe des Zweit-Sensors abgeschätzt wird, dass beide Sensoren vom gleichen Sensortyp sind und dass ein Zeitpunkt für eine Kalibrierung oder das Erreichen eines Austauschzustandes als von der Alterung abhängige Größe abgeschätzt wird.

In einer Ausgestaltung ist vorgesehen, dass der Sensor so lange kalibriert wird, bis es zu einem Funktionsausfall des Sensors kommt. In dieser Ausgestaltung werden Referenzdaten über einen gesamten Lebenszyklus eines Sensors an der Messstelle erzeugt bzw. gewonnen.

In einer alternativen oder ergänzenden Ausgestaltung wird der Sensor so lange kalibriert, bis mit einer Kalibrierung verbundene Daten außerhalb eines vorgebbaren Toleranzbandes liegen. Wird beispielsweise bei einer Kalibrierung ermittelt, dass die Parameter außerhalb eines Toleranzbandes liegen oder dass die zu erreichende Genauigkeit trotz einer Kalibrierung außerhalb eines Toleranzbandes liegt oder dass bereits die Zeitdauer für eine Kalibrierung außerhalb eines Toleranzbandes liegt, so wird die Reihe der Kalibrierungen mit diesem Sensor beendet. Dies bezieht sich also auf den Fall, dass der Sensor zwar an sich noch funktionstüchtig ist, dass er durch Alterungserscheinungen aber nicht mehr den Anforderungen entspricht, die durch mindestens ein Toleranzband definiert bzw. umgesetzt werden.

Erfindungsgemäß wird ein Zeitpunkt für eine Kalibrierung oder das Erreichen eines Austauschzustandes als von der Alterung abhängige Größe abgeschätzt.

In einer sich daran anschließenden Ausgestaltung wird nur ein solcher Zeitpunkt für den von dem Sensor unterschiedlichen Sensor abgeschätzt, der zeitlich innerhalb eines von den Kalibrier-Zeitpunkten des Sensors aufgespannten Zeitraums liegt.

Für die Abschätzung wird insbesondere nur eine Interpolation anhand der hinterlegten Referenzdaten und des damit verbundenen Zeitbereichs vorgenommen. Eine Extrapolation auf Zeitpunkte, für die keine Referenzdaten vorhanden sind, wird insbesondere vermieden.

Umfassen also beispielsweise die Referenzdaten einen Lebenszeitraum von x Monaten des Sensors, so werden für einen anderen Sensor auch nur solche Zeitpunkte abgeschätzt, die bis zu einer Lebensdauer des anderen Sensors von x Monaten reichen.

Werden also beispielsweise Referenzdaten gewonnen, die zwischen der Inbetriebnahme des Sensors als Nullpunkt und einem Zeitpunkt T liegen und die daher eine Lebensdauer eines Sensors von T Zeiteinheiten beschreiben, so werden auch nur Zeitpunkte für einen anderen Sensor abgeschätzt, die innerhalb dieser T Zeiteinheiten liegen.

Oder mit anderen Worten: Beziehen sich die Referenzdaten des Sensors auf eine Zeitdauer, die kleiner ist als die Zeitdauer, die ein zum Sensor unterschiedlicher Sensor in Betrieb ist, so erfolgen für letzteren Sensor keine weiteren Abschätzungen mehr, da die - z. B. nur über eine Extrapolation -abschätzbaren Zeitpunkte außerhalb des Zeitrahmens der Referenzdaten liegen.

Die Abschätzung der Zeitpunkte basiert daher auf gemessenen bzw. abgespeicherten und nicht auf durch unterschiedliche mathematische Modelle gewonnenen Extrapolationen.

In einer Ausgestaltung werden auch Interpolationen zugelassen, insofern aus den einzelnen Kalibrier-Zeitpunkten mit mathematischen Modellen kontinuierliche Zeitverläufe bzw. Kurven erzeugt werden.

In einer Ausgestaltung ist vorgesehen, dass an der Messstelle der Sensor durch einen Ersatz-Sensor ausgetauscht wird.

In einer sich daran ggf. anschließenden Ausgestaltung wird der Ersatz-Sensor an vorgebbaren Kalibrier-Zeitpunkten kalibriert und wird jeweils mindestens ein mit der Kalibrierung verbundener Parameter mit den Referenzdaten des Sensors zu Sensortyp-Referenzdaten der Messstelle kombiniert. Durch den Ersatz-Sensor werden also weitere Referenzdaten gewonnen und mit den Referenzdaten des Sensors zu Sensortyp-Referenzdaten für die Messstelle kombiniert.

Erfindungsgemäß ist vorgesehen, dass an einer Zweit-Messstelle von mindestens einem Zweit-Sensor mindestens eine Messgröße ermittelt wird und dass ausgehend von den Referenzdaten des Sensors mindestens eine von der Alterung abhängige Größe des Zweit-Sensors abgeschätzt wird. In dieser Ausgestaltung dient das Verfahren also insgesamt dem Betreiben von zwei Messstellen.

Die Messstelle und die Zweit-Messstelle befinden sich insbesondere an unterschiedlichen Stellen einer Prozessanlage.

Dabei herrschen in einer Ausgestaltung an der Messstelle und der Zweit-Messstelle gleiche oder annährend gleiche Prozessbedingungen (z. B. Temperatur, Druck, Feuchtigkeit usw.).

In einer weiteren Ausgestaltung ist zumindest periodisch wiederkehrend eine Gleichheit der Prozessbedingungen gegeben.

In einer alternativen Ausgestaltung werden die Referenz oder die Sensortyp-Referenzdaten in Bezug auf die Prozessbedingungen von der Messstelle auf die Zweit-Messstelle umgerechnet bzw. entsprechend skaliert.

Weiterhin bezieht sich die Erfindung auf ein Verfahren zum Betreiben einer Zweit-Messstelle, wobei an der Zweit-Messstelle von mindestens einem Zweit-Sensor mindestens eine Messgröße ermittelt wird. Dabei ist vorgesehen, dass ausgehend von Referenzdaten eines an einer von der Zweit-Messstelle unterschiedlichen Messstelle mindestens eine Messgröße ermittelnden Sensors mindestens eine von der Alterung abhängige Größe des Zweit-Sensors abgeschätzt wird, dass beide Sensoren vom gleichen Sensortyp sind und dass ein Zeitpunkt für eine Kalibrierung oder das Erreichen eines Austauschzustandes als von der Alterung abhängige Größe abgeschätzt wird.

Die oben getätigten Aussagen und Ausgestaltungen beziehen sich dabei entsprechend hier auch auf die Gewinnung der Referenzdaten bzw. ggf. der Sensortyp-Referenzdaten, die ebenfalls auf den Referenzdaten fußen.

Beim Betreiben der Zweit-Messstelle ergibt sich hier der Vorteil, dass bereits auf vorhandene Referenzdaten einer anderen Messstelle zurückgegriffen werden kann. Hier kann also beispielsweise die Zweit-Messstelle neu erzeugt werden und bereits mit dem erstmalig installierten Zweit-Sensor ist bereits ein vorausschauender Betrieb anhand der an der Messstelle gewonnenen Daten möglich.

In einer Ausgestaltung ist vorgesehen, dass mit dem Sensor der pH-Wert, der Sauerstoffgehalt, der Chlorgehalt, die Leitfähigkeit, der Ozongehalt, der Wasserstoffperoxidgehalt, der Gehalt an freiem Chlor, der Gehalt an residualem Chlor, die Trübung oder / und der Feststoffanteil als Messgröße ermittelt wird. Entsprechend der Art des Sensors sind insbesondere auch der Ersatz-Sensor und/oder der Zweit-Sensor ausgeführt.

Die Erfindung sei mit anderen Worten noch einmal beschrieben:
Herrschen in Prozessanlagen an den jeweiligen Messstellen weitestgehend typisch prozesstechnisch konstante Bedingungen, so altern die Messsensoren an den jeweiligen Messstellen über ihre Lebensdauer aufgrund ähnlich auftretender Alterungseffekte.

Daher wird an der Messstelle mit dem Sensor ein typischer oder charakteristischer Verlauf der Alterung aufgenommen. Dies geschieht durch die Registrierung der Parameter der Kalibrierungen. Diese Parameter verändern sich über der Lebensdauer.

Die Referenzdaten werden in einer Ausgestaltung als Basis beispielsweise für die Abschätzung der Lebensdauer weiterer Sensoren an der Messstelle oder an der Zweit-Messstelle genutzt.

Werden auch bei - an der vorzugsweise gleichen Messstelle - nachfolgenden Ersatz-Sensoren die Parameter bei Kalibrierungen aufgenommen, so können diese mit den bestehenden Referenzdaten kombiniert, z. B. gemittelt werden, um die Genauigkeit der Vorhersage zu verbessern.

In einem weiteren Schritt ist dieses Modell skalierbar, so dass an einer Zweit-Messstelle mit anderen Prozessbedingungen die Referenzdaten der Messstelle verwendet bzw. für die Unterschiede der Prozessbedingungen skaliert werden.

Die Speicherung der Referenzdaten, die Abschätzungen oder Anpassungen der Referenz- zu Sensortyp-Referenzdaten erfolgt dabei je nach Ausgestaltung in den Sensoren, in sogenannten Transmittern bzw. Messumformern, in einer Leitwarte oder in externen Geräten.

Im Einzelnen gibt es eine Vielzahl von Möglichkeiten, die erfindungsgemäßen Verfahren auszugestalten und weiterzubilden. Dazu wird verwiesen einerseits auf die den Patentansprüchen 1 und 4 nachgeordneten Patentansprüche, andererseits auf die folgende Beschreibung von Ausführungsbeispielen in Verbindung mit der Zeichnung. In der Zeichnung zeigen
- Fig. 1: einen Schnitt durch eine schematische Darstellung eines Teils einer Prozessanlage,
- Fig. 2: zwei Zeitstrahlen mit unterschiedlichen Zeitpunkten und
- Fig. 3: ein Ablaufdiagramm für ein Verfahren zum Betreiben einer Messstelle und einer Zweit-Messstelle.

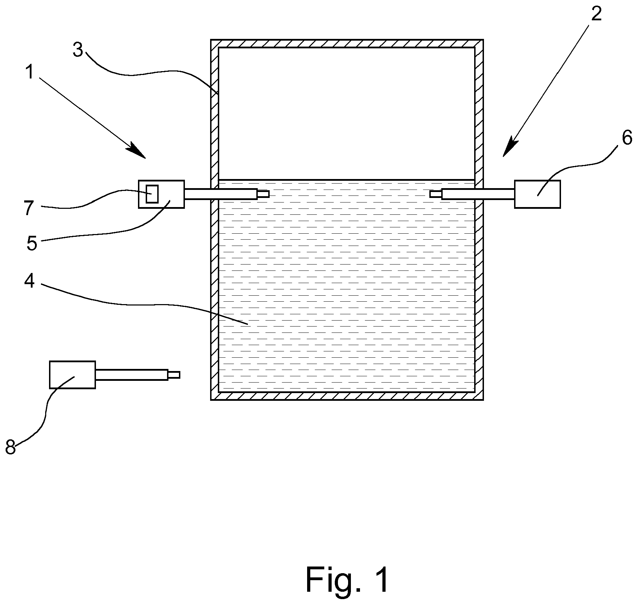

In der Fig. 1 ist schematisch ein Teil einer Prozessanlage mit einer ersten Messstelle 1 und einer Zweit-Messstelle 2 dargestellt. Die beiden Messstellen 1, 2 unterscheiden sich hinsichtlich ihrer Position an dem Behälter 3, in dem sich ein Medium 4 - hier eine Flüssigkeit - befindet.

Um den pH-Wert des Mediums 4 zu bestimmen, ist an der Messstelle 1 ein Sensor 5 und an der Zweit-Messstelle 2 ein Zweit-Sensor 6 angebracht. Beide Sensoren 5, 6 sind insbesondere vom gleichen Sensor-Typ.

Der Sensor 5 wird zu unterschiedlichen Kalibrier-Zeitpunkten kalibriert und die damit verbundenen Parameter werden als Referenzdaten in einer Speichereinheit 7 abgespeichert. Die Parameter beziehen sich beispielsweise auf die Werte, über die aus der gemessenen elektrischen Spannung auf den vorliegenden pH-Wert umgerechnet wird. Aus den Parameterwerten ergibt sich ein Maßstab für die Lebensdauer bzw. für den Alterungszustand des Sensors 5.

Die aus den durchgeführten Kalibrierungen gewonnenen Referenzdaten des Sensors 5 werden dabei auch für die Abschätzung von relevanten Zeitpunkten des Zweit-Sensors 6 an der Zweit-Messstelle 2 verwendet.

Die Verwendung der Referenzdaten erfolgt hier unter der Rahmenbedingung des Vorliegens von im Wesentlichen gleichen oder vergleichbaren oder zumindest zeitweise oder periodisch vergleichbaren bzw. gleichen Prozessbedingungen an der Messstelle 1 und der Zweit-Messstelle 2. In einer alternativen Ausgestaltung erfolgt eine Anpassung der Referenzdaten im Hinblick auf unterschiedliche Prozessbedingungen.

Ist der Sensor 5 nicht mehr funktionsfähig, so wird er durch einen Ersatz-Sensor 8 ausgetauscht, der vom gleichen Sensortyp wie der Sensor 5 ist. Für die Alterungsbetrachtung des Ersatz-Sensors 8 werden dann die Referenzdaten des vorher installierten Sensors 5 verwendet.

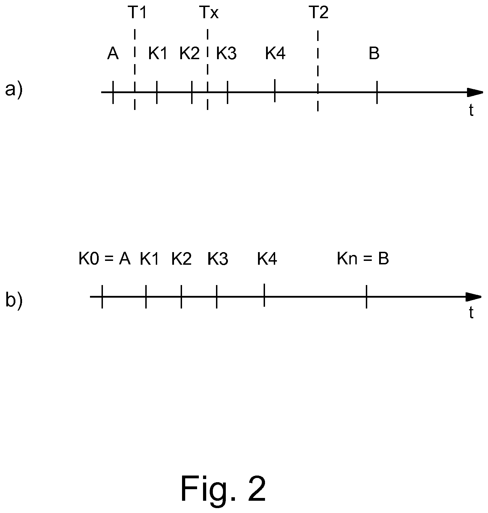

In der Fig. 2 a) und b) wird schematisch anhand zweier Zeitachsen für die Zeit t erläutert, wie gemäß einer Ausgestaltung die Kalibrier-Zeitpunkte und die Zeitpunkte als abgeschätzte Größe zusammenhängen.

Der in der Fig. 1 an der Messstelle befestigte Sensor wird zum Zeitpunkt A in Betrieb genommen und wird zum Zeitpunkt B infolge eines Funktionsausfalls wieder entfernt. Der Funktionsausfall kann sich dabei auf eine vollständige Beendigung der Messfähigkeit oder auch auf eine Messunsicherheit bedingt durch Alterungseffekte jenseits einer vorgebbaren Grenze beziehen.

Auf der Zeitachse der Fig. 2 a) wird zu den Zeitpunkten K1 bis K4 der Sensor kalibriert und werden die damit verbundenen Parameter als Referenzdaten des Sensors bezogen auf die Messestelle abgespeichert.

Im Fall der Zeitachse der Fig. 2 b) wird zu zwei weiteren Zeitpunkten K0 und Kn der Sensor kalibriert. Dies ist zum einen der Zeitpunkt der Inbetriebnahme des Sensors, weshalb K0 und A zusammenfallen. Zum anderen ist dies der Kalibrier-Zeitpunkt Kn, an dem erkannt wird, dass der Sensor auszutauschen ist. Daher fallen die Zeitpunkte Kn und B zusammen.

Soll - im Fall der Fig. 2 a) - ausgehend von den Referenzdaten beispielsweise für den Ersatz-Sensor, der sich an der gleichen Messstelle befindet, ein Zeitpunkt Tx abgeschätzt werden, wann eine Kalibrierung erforderlich ist, so ist vorgesehen, dass sich dies nur auf einen Zeitpunkt zwischen K1 und K4 relativ zum Zeitpunkt A bzw. zwischen A und B bezieht.

Ist also beispielsweise der Ersatz-Sensor erst eine so kurze Zeit T1 zwischen den Punkten A - als seinem Nullpunkt der Installation und Inbetriebnahme - und K1 installiert, so kann der früheste abgeschätzte Zeitpunkt für eine Kalibrierung des Ersatz-Sensors nach dem Zeitpunkt K1 - also z. B. Tx - liegen.

Andererseits sind auch keine Zeitpunkte für eine Kalibrierung abschätzbar, die zwischen den Zeitpunkten K4 und B liegen, die also später als der Zeitraum der Referenzdaten wären. Ist also der Ersatz-Sensor länger als die Zeitdauer zwischen A und K4 - z. B. bis zum Zeitpunkt T2 - in Betrieb, so sind keine Zeitpunkte abschätzbar, da diese auf einer Extrapolation beruhen würden.

Im Gegensatz dazu erlauben die Kalibrier-Zeitpunkte der Fig. 2 b) einen vollständigen Abschätzungsbereich zwischen der Installation (Zeitpunkt A) und der De-Installation (Zeitpunkt B) des Sensors.

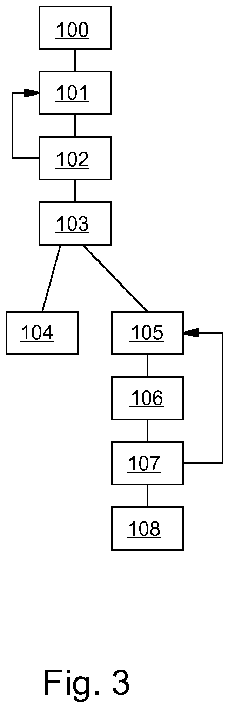

Ein Ablauf für ein Betreiben der Messstelle und der Zweit-Messstelle der Fig. 1 ist der Fig. 3 zu entnehmen.

Im Schritt 100 wird der Sensor an der Messstelle in Betrieb genommen. Im Schritt 101 wird der Sensor kalibriert und im Schritt 102 werden die damit verbundenen Parameter - bzw. mindestens ein damit verbundener Parameter - abgespeichert. Daran schließen sich weitere Kalibrierungen 101 an, bis der Sensor von der Messstelle entfernt und im Schritt 103 durch einen Ersatz-Sensor ersetzt wird.

Im Schritt 104 wird der Ersatz-Sensor mit den Referenzdaten des Sensors betrieben, so dass Zeitpunkte für Kalibrierungen oder für den Austausch des Ersatz-Sensors abgeschätzt werden.

Im alternativen Schritt 105 wird der Ersatz-Sensor einer Kalibrierung unterzogen, wobei die damit verbundenen Parameter im Schritt 106 abgespeichert werden und im Schritt 107 die Referenzdaten des Sensors mit den Daten des Ersatz-Sensors zu Sensortyp-Referenzdaten der Messstelle verarbeitet werden. Bei gleichen Kalibrier-Zeitpunkten - bezogen auf die Betriebsdauer - werden beispielsweise die jeweiligen Parameter gemittelt.

Die Sensortyp-Referenzdaten des Sensortyps, zu dem der Sensor und der Ersatz-Sensor gehören, sind daher insgesamt genauer oder weisen mehr Kalibrier-Zeitpunkte als die zunächst gewonnenen Referenzdaten auf.

Auch dies wird für eine vorgebbare Zeitdauer oder auch bis zum Erreichen des Funktionsausfalls des Ersatz-Sensors wiederholt.

Hier werden anschließend im Schritt 108 die Sensortyp-Referenzdaten für den Zweit-Sensor an der Zweit-Messstelle verwendet.

## Patentansprüche

1. Verfahren zum Betreiben einer Messstelle (1), wobei an der Messstelle (1) von mindestens einem kalibrierbaren Sensor (5) mindestens eine Messgröße ermittelt wird, wobei der Sensor (5) an vorgebbaren Kalibrier-Zeitpunkten kalibriert wird und wobei jeweils mindestens ein mit der Kalibrierung verbundener Parameter als Teil von Referenzdaten des Sensors (5) hinterlegt wird,
**dadurch gekennzeichnet,**
**dass** an einer Zweit-Messstelle (2) von einem Zweit-Sensor (6) mindestens eine Messgröße ermittelt wird und dass ausgehend von den Referenzdaten des Sensors (5) mindestens eine von der Alterung abhängige Größe des Zweit-Sensors (6) abgeschätzt wird,
**dass** beide Sensoren (5, 6) vom gleichen Sensortyp sind und
**dass** ein Zeitpunkt für eine Kalibrierung oder das Erreichen eines Austauschzustandes als von der Alterung abhängige Größe abgeschätzt wird.

2. Verfahren nach Anspruch 1, **dadurch gekennzeichnet, dass** der Sensor (5) so lange kalibriert wird, bis es zu einem Funktionsausfall des Sensors (5) kommt und/oder bis mit einer Kalibrierung verbundene Daten außerhalb eines vorgebbaren Toleranzbandes liegen.

3. Verfahren nach Anspruch 1 oder 2, **dadurch gekennzeichnet, dass** für den Zweit-Sensor (6) nur ein solcher Zeitpunkt abgeschätzt wird, der zeitlich innerhalb eines von den Kalibrier-Zeitpunkten des Sensors (5) aufgespannten Zeitraums liegt.

4. Verfahren zum Betreiben einer Zweit-Messstelle (2), wobei an der Zweit-Messstelle (2) von mindestens einem Zweit-Sensor (6) mindestens eine Messgröße ermittelt wird,
**dadurch gekennzeichnet,**
**dass** ausgehend von Referenzdaten eines an einer von der Zweit-Messstelle (2) unterschiedlichen Messstelle (1) mindestens eine Messgröße ermittelnden Sensors (5) mindestens eine von der Alterung abhängige Größe des Zweit-Sensors (6) abgeschätzt wird,
**dass** beide Sensoren (5, 6) vom gleichen Sensortyp sind und
**dass** ein Zeitpunkt für eine Kalibrierung oder das Erreichen eines Austauschzustandes als von der Alterung abhängige Größe abgeschätzt wird.

5. Verfahren nach einem der Ansprüche 1 bis 4, **dadurch gekennzeichnet, dass** mit dem Sensor (5) der pH-Wert, der Sauerstoffgehalt, der Chlorgehalt, die Leitfähigkeit, der Ozongehalt, der Wasserstoffperoxidgehalt, der Gehalt an freiem Chlor, der Gehalt an residualem Chlor, die Trübung oder / und der Feststoffanteils als Messgröße ermittelt wird.

## Claims

1. Method for operating a measuring point (1), wherein at least one measured variable is determined at the measuring point (1) by at least one calibration-capable sensor (5), wherein the sensor (5) is calibrated at predefinable calibration times and at least one parameter connected to the calibration is stored, in each case, as part of reference data of the sensor (5),
**characterized in**
**that** at least one measured variable is determined at a second measuring point (2) by a second sensor (6) and that at least one variable of the second sensor (6), which depends on aging, is estimated from the reference data of the sensor (5),
**that** both sensors (5, 6) are of the same sensor type and
**that** a time for a calibration or of reaching a replacement state is estimated as a quantity dependent on aging.

2. Method according to claim 1, **characterized in that** the sensor (5) is calibrated until a functional failure of the sensor (5) occurs and/or until data connected to a calibration lie outside a predeterminable tolerance band.

3. Method according to claim 1 or 2, **characterized in that** only such a time is estimated for the second sensor (6) that lies within a time period spanned by the calibration times of the sensor (5).

4. Method for operating a second measuring point (2), wherein at least one measured variable is determined at the second measuring point (2) by at least one second sensor (6),
**characterized in**
**that** at least one measured variable dependent on the aging of the second sensor (6) is estimated based on reference data of sensor determining at least one measured variable at a measuring point (1) differing from the second measuring point (2)
**that** both sensors (5, 6) are of the same sensor type and
**that** a time for a calibration or of reaching a replacement state is estimated as a quantity dependent on aging.

5. Method according to any one of claims 1 to 4, **characterized in that** the pH value, the oxygen content, the chlorine content, the conductivity, the ozone content, the hydrogen peroxide content, the content of free chlorine, the content of residual chlorine, the turbidity and/or the solids content is determined as measured variable with the sensor (5).

## Revendications

1. Procédé de mise en oeuvre d'un point de mesure (1), dans lequel au moins une grandeur de mesure est déterminée au point de mesure (1) par au moins un capteur étalonnable (5), dans lequel le capteur (5) est étalonné à des instants d'étalonnage prédéfinis et dans lequel au moins un paramètre lié à l'étalonnage est mémorisé en tant que partie de données de référence du capteur (5),
**caractérisé en ce qu'**au moins une grandeur de mesure est déterminée en un second point de mesure (2) par un second capteur (6) et **en ce qu'**au moins une grandeur du second capteur (6) dépendant du vieillissement est estimée sur la base des données de référence du capteur (5),
**en ce que** les deux capteurs (5, 6) sont des capteurs du même type et
**en ce qu'**un instant lors duquel un étalonnage effectué ou lors duquel un état de remplacement est atteint est estimé en tant que grandeur dépendant du vieillissement.

2. Procédé selon la revendication 1, **caractérisé en ce que** le capteur (5) est étalonné jusqu'à ce qu'une défaillance fonctionnelle du capteur (5) se produise et/ou jusqu'à ce que les données associées à un étalonnage se situent en dehors d'une bande de tolérance pouvant être prédéfinie.

3. Procédé selon la revendication 1 ou 2, **caractérisé en ce que**, pour le second capteur (6), on estime uniquement un instant qui se situe à l'intérieur d'une période de temps correspondant aux instants d'étalonnage du capteur (5).

4. Procédé de mise en oeuvre d'un second point de mesure (2), dans lequel au moins une grandeur de mesure est déterminée au second instant de mesure (2) par au moins un second capteur (6),
**caractérisé en ce qu'**au moins une grandeur mesurée du second capteur (6) est estimée à partir des données de référence d'un capteur (5) déterminant au moins une grandeur de mesure en un point de mesure (1) différent du second point de mesure (2),
**en ce que** les deux capteurs (5, 6) sont des capteurs du même type et
**en ce qu'**un instant lors duquel un étalonnage est effectué ou lors duquel un état de remplacement est atteint est estimé en tant que grandeur dépendant du vieillissement.

5. Procédé selon les revendications 1 à 4, **caractérisé en ce que** la valeur du pH, la teneur en oxygène, la teneur en chlore, la conductivité, la teneur en ozone, la teneur en peroxyde d'hydrogène, la teneur en chlore libre, la teneur en chlore résiduel, la turbidité et/ou la teneur en matières solides est/sont déterminée(s) en tant que grandeur de mesure au moyen du capteur (5).
